# EUROPEAN PATENT SPECIFICATION

(11) **EP 3 629 908 B1**
(45) Date of publication and mention of the grant of the patent: **24.04.2024**
(21) Application number: 18806926.4
(22) Date of filing: 23.05.2018
(51) Int. Cl.: A61B 5/021, A61B 5/00

(54) **SYSTOLIC PRESSURE CALIBRATION OF A CONTINUOUS BLOOD PRESSURE MEASUREMENT SYSTEM USING THE VOLUME-CLAMP METHOD**
SYSTOLISCHE DRUCKKALIBRIERUNG EINES SYSTEMS ZUR KONTINUIERLICHEN VOLUMENKLEMM-BLUTDRUKMESSUNG
ÉTALONNAGE DE PRESSION SYSTOLIQUE D'UN SYSTÈME DE MESURE CONTINUE DE PRESSION ARTÉRIELLE À SERRAGE DE VOLUME

(30) Priority: 24.05.2017 US 201762510351 P; 21.05.2018 US 201815984684
(43) Date of publication of application: 08.04.2020
(73) Proprietor: Edwards Lifesciences Corporation, Irvine, CA 92614 (US)
(72) Inventor: WESTERHOF, Berend, Irvine CA 92614 (US); SCHRAA, Olaf, Irvine CA 92614 (US); VAN GROENINGEN, Christianus Joannes, Jozef Eugène, Irvine CA 92614 (US); LI, Peiyuan, Irvine CA 92614 (US); VAN GOUDOEVER, Jeroen, Irvine CA 92614 (US)
(74) Representative: Eisenführ Speiser
(86) International application number: PCT/US2018/034084
(87) International publication number: WO 2018/217861

(56) References cited:
- EP-A1- 0 444 934
- WO-A1-2004/100783
- WO-A1-2005/018444
- WO-A1-2010/063976
- WO-A1-2016/146356
- US-A- 4 105 021
- US-A- 4 475 554
- US-A1- 2012 245 471
- US-A1- 2015 080 669
- US-B1- 6 647 287

## Description

### BACKGROUND

### Field

The invention relates to non-invasive blood pressure measurement using a finger cuff and a volume clamp method and that utilizes systolic pressure calibration.

### Relevant Background

Volume clamping is a technique for non-invasively measuring blood pressure in which pressure is applied to a subject's finger in such a manner that arterial pressure may be balanced by a time varying pressure to maintain a constant arterial volume. In a properly fitted and calibrated system, the applied time varying pressure is equal to the arterial blood pressure in the finger. The applied time varying pressure may be measured to provide a reading of the patient's arterial blood pressure.

This may be accomplished by a finger cuff that is arranged around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter and indicative of the pressure in the artery.

In the finger cuff implementation, by inflating the bladder in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder such that the diameter of the finger artery is kept constant, the blood pressure may be monitored in very precise detail as the pressure in the inflatable bladder is directly linked to the blood pressure.

In a typical present day finger cuff implementation, a volume clamp system is used with the finger cuff. The volume clamp system typically includes a pressure generating system and a regulating system that includes: a pump, a valve, and a pressure sensor in a closed loop feedback system that are used in conjunction with the measurement of the arterial volume. To accurately measure blood pressure, the feedback loop provides sufficient pressure generating and releasing capabilities to match the pressure oscillations of the subject's blood pressure.

Calibrating a volume clamp system with known methods may be difficult and/or cumbersome. In the prior art, an algorithm has been described that switches between volume clamp blood pressure measurement and periods with a constant cuff pressure, during which the shape of plethysmographic waveform is analyzed to determine the plethysmogram corresponding to an unloaded artery (EP 0080778 B1). The unloaded volume state can also found by introducing a high frequency disturbance on the pressure signal during volume clamp mode (EP 0284095 B1). By analyzing the corresponding plethysmographic changes the volume with maximal dynamic 'GefaBkomplianz' compliance can be located. Another method to calibrate a volume clamp blood pressure measurement is to first find an approximation of the unloaded volume (e.g., by determining at which cuff pressure level the beat-to-beat amplitude of the plethysmogram is largest) and then to perform a calibration with an independent reference blood pressure (such as an oscillometric blood pressure measurement with upper arm cuff). An upper arm cuff can also be used to determine systolic pressure if the arm cuff is first inflated to a pressure level above systolic pressure, and then is gradually reduced, and by subsequently determining at which cuff pressure the heart pulsations can be first detected in the finger blood pressure signal or plethysmogram (U.S. Patent No. 5,746,698 A). Due to a gradual pressure decay and pressure waveform distortion towards the periphery the finger blood pressure may deviate from arm cuff pressure, and currently there is no good way to calibrate a finger blood pressure volume clamp measurement with sufficient accuracy. Document WO 2016/146356 A1 relates to the measurement of blood pressure using a volume-clamp blood pressure method, and in particular relates to improving the calibration of a system that uses a volume-clamp method. Document WO 2010/063976 A1 relates to a non-invasive method for measuring systolic blood pressure using two arterial occluding members placed on the same finger. Document WO 2005/018444 A1 discloses a non-invasive measurement of blood pressure with a frequency that approximates a continuous measurement.

### SUMMARY OF THE INVENTION

A method for monitoring blood pressure of a patient in accordance with the invention is defined in claim 1. Embodiments of the method of the invention are defined in claims 2 to 9. A system for monitoring blood pressure of a patient in accordance with the invention is defined in claim 10. Embodiments of the system of the invention are defined in claims 11 to 14. A non-transitory computer-readable medium in accordance with the invention is defined in claim 15.

Embodiments of the invention relate to a method for measuring systolic arterial blood pressure of a patient utilizing a finger cuff including an inflatable bladder and a pulsatility sensor that detects arterial pulsatility in a finger of the patient. The method comprises, among other features: placing the finger cuff around the finger of the patient; inflating the bladder of the finger cuff until the finger cuff applies a first pressure to the finger, the first pressure being greater than the systolic pressure to be measured; deflating the bladder of the finger cuff until the finger cuff applies a second pressure to the finger, the second pressure being less than the systolic pressure to be measured; monitoring the arterial pulsatility in the finger with the pulsatility sensor; and obtaining a measurement of the systolic arterial blood pressure based on pressure applied to the finger by the finger cuff and the arterial pulsatility in the finger. As will be described, various techniques are utilized to obtain the measurement of the systolic arterial blood pressure which is used to calibrate the arterial blood pressure measurement system.

### BRIEF DESCRIPTION OF THE DRAWINGS

FIG. 1 is a diagram of an example of a blood pressure measurement system.
FIG. 2 is a block diagram illustrating an example environment in which embodiments of the invention may be practiced.
FIG. 3 is a block diagram illustrating example control circuitry.
FIG. 4 is a flowchart illustrating an example method for measuring a finger systolic arterial blood pressure.
FIG. 5 is a diagram illustrating various measurement traces according to embodiments.
FIG. 6 is a block diagram illustrating an example environment in which embodiments of the invention may be practiced.

### DETAILED DESCRIPTION

Embodiments of the invention relate to a method for measuring finger systolic arterial blood pressure in accordance with claim 1, comprising, among other features: placing a finger cuff around a finger of a patient, the finger cuff comprising an inflatable bladder and a pulsatility sensor that detects arterial pulsatility in the finger; inflating the bladder of the finger cuff until the finger cuff applies a first pressure to the finger, the first pressure being greater than the systolic pressure to be measured; deflating the bladder of the finger cuff until the finger cuff applies a second pressure to the finger, the second pressure being less than the systolic pressure to be measured; monitoring the arterial pulsatility in the finger with the pulsatility sensor; and obtaining a measurement of the finger systolic arterial blood pressure based on pressure applied to the finger by the finger cuff and the arterial pulsatility in the finger. As will be described, various techniques are utilized to obtain the measurement of the systolic arterial blood pressure which is used to calibrate the arterial blood pressure measurement system.

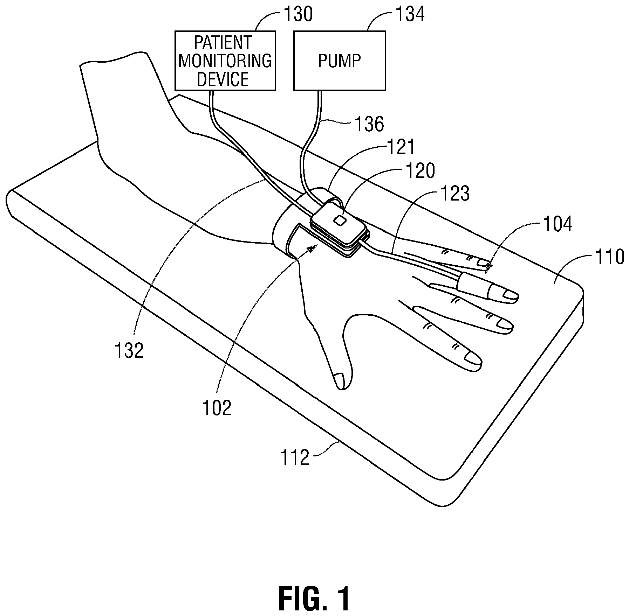

With reference to FIG. 1, an example of an environment in which a finger cuff 104 may be implemented will be described. As an example, a blood pressure measurement system 102 that includes a finger cuff 104 that may be attached to a patient's finger and a blood pressure measurement controller 120 that may be attached to the patient's body (e.g., a patient's wrist or hand) is shown. The blood pressure measurement system 102 may further be connected to a patient monitoring device 130, and, in some embodiments, a pump 134. Further, finger cuff 104 may include a bladder (not shown) and an LED-PD pair (not shown), which are conventional for finger cuffs.

In one embodiment, the blood pressure measurement system 102 may include a pressure measurement controller 120 that includes: a small internal pump, a small internal valve, a pressure sensor, and control circuity. In this embodiment, the control circuitry may be configured to: control the pneumatic pressure applied by the internal pump to the bladder of the finger cuff 104 to replicate the patient's blood pressure based upon measuring the pleth signal received from the LED-PD pair of the finger cuff 104. Further, the control circuitry may be configured to: control the opening of the internal valve to release pneumatic pressure from the bladder; or the internal valve may simply be an orifice that is not controlled. Additionally, the control circuitry may be configured to: measure the patient's blood pressure by monitoring the pressure of the bladder based upon the input from a pressure senor, which should be the same as patient's blood pressure, and may display the patient's blood pressure on the patient monitoring device 130.

In another embodiment, a conventional pressure generating and regulating system may be utilized, in which, a pump 134 is located remotely from the body of the patient. In this embodiment, the blood pressure measurement controller 120 receives pneumatic pressure from remote pump 134 through tube 136 and passes on the pneumatic pressure through tube 123 to the bladder of finger cuff 104. Blood pressure measurement device controller 120 may also control the pneumatic pressure (e.g., utilizing a controllable valve) applied to the finger cuff 104 as well as other functions. In this example, the pneumatic pressure applied by the pump 134 to the bladder of finger cuff 104 to replicate the patient's blood pressure based upon measuring the pleth signal received from the LED-PD pair of the finger cuff 104 and measuring the patient's blood pressure by monitoring the pressure of the bladder may be controlled by the blood pressure measurement controller 120 and/or a remote computing device and/or the pump 134 and/or the patient monitoring device 130. In some embodiments, a blood pressure measurement controller 120 is not used at all and there is simply a connection from the tube 123 to finger cuff connector 122 from a remote pump 134 including a remote pressure regulatory system, and all processing for the pressure generating and regulatory system, data processing, and display is performed by a remote computing device.

Continuing with this example, as shown in FIG. 1, a patient's hand may be placed on the face 110 of an arm rest 112 for measuring a patient's blood pressure with the blood pressure measurement system 102. The blood pressure measurement controller 120 of the blood pressure measurement system 102 may be coupled to a bladder of the finger cuff 104 in order to provide pneumatic pressure to the bladder for use in blood pressure measurement. Blood pressure measurement controller 120 may be coupled to the patient monitoring device 130 through a power/data cable 132. Also, in one embodiment, as previously described, in a remote implementation, blood pressure measurement controller 120 may be coupled to a remote pump 134 through tube 136 to receive pneumatic pressure for the bladder of the finger cuff 104. The patient monitoring device 130 may be any type of medical electronic device that may read, collect, process, display, etc., physiological readings/data of a patient including blood pressure, as well as any other suitable physiological patient readings. Accordingly, power/data cable 132 may transmit data to and from patient monitoring device 130 and also may provide power from the patient monitoring device 130 to the blood pressure measurement controller 120 and finger cuff 104.

As can be seen in FIG. 1, in one example, the finger cuff 104 may be attached to a patient's finger and the blood pressure measurement controller 120 may be attached on the patient's hand or wrist with an attachment bracelet 121 that wraps around the patient's wrist or hand. The attachment bracelet 121 may be metal, plastic, Velcro, etc. It should be appreciated that this is just one example of attaching a blood pressure measurement controller 120 and that any suitable way of attaching a blood pressure measurement controller to a patient's body or in close proximity to a patient's body may be utilized and that, in some embodiments, a blood pressure measurement controller 120 may not be used at all. It should further be appreciated that the finger cuff 104 may be connected to a blood pressure measurement controller described herein, or a pressure generating and regulating system of any other kind, such as a conventional pressure generating and regulating system that is located remotely from the body of the patient (e.g., a pump 134 located remotely from a patient). Any kind of pressure generating and regulating system that can be used, including but not limited to the blood pressure measurement controller, may be described simply as a pressure generating and regulating system. As a further example, in some embodiments, there may be no blood pressure measurement controller, at all, and a remote pump 134 that is controlled remotely may be directly connected via a tube 136 and 123 to finger cuff 104 to provide pneumatic pressure to the finger cuff 104.

Calibrating a volume clamp method-based non-invasive finger arterial blood pressure measurement system, such as the system illustrated in Figure 1, may be difficult and/or cumbersome. Systolic pressure has always been the most difficult pressure to obtain precisely from the finger arterial pressure.

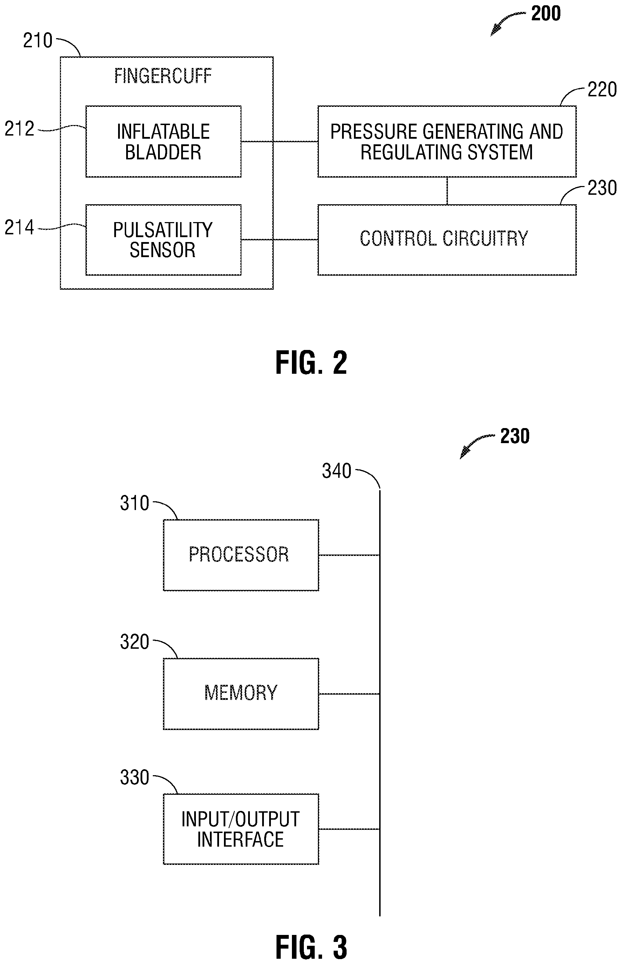

Referring to Figure 2, a block diagram illustrating an example environment 200 in which embodiments of the invention may be practiced is shown. The environment as illustrated is not claimed here. The finger cuff 210 may comprise an inflatable bladder 212 and an arterial pulsatility sensor 214. The inflatable bladder 212 may be pneumatically connected to a pressure generating and regulating system 220. The pressure generating and regulating system 220 may generate, measure, and regulate pneumatic pressure that inflates or deflates the bladder 212, and may comprise such elements as a pump, a valve, a sensor, control circuitry, and/or other suitable elements. When the bladder 212 is inflated, the finger cuff 210 applies a pressure to the finger. The pressure applied to the finger by the finger cuff 210 may be the same as the pneumatic pressure in the bladder 212.

In one embodiment, the arterial pulsatility sensor 214 may comprise a plethysmograph. The plethysmograph may make continuous volumetric measurements (or plethysmogram) of arterial blood flows within the finger. Thus, pulsatility in the finger may be detected based on the plethysmogram. In one embodiment, the plethysmograph may comprise a light-emitting diode (LED) - photodiode pair. The LED may be used to illuminate the finger skin and light absorption or reflection may be detected with the photodiode. Therefore, the plethysmogram may be generated based on the signal received from the photodiode.

The pressure generating and regulating system 220 and the arterial pulsatility sensor 214 may be connected to a control circuitry 230. The control circuitry 230 may instruct the pressure generating and regulating system 220 to inflate or deflate the bladder 212 based on a pressure setting, may receive pulsatility information from the pulsatility sensor 214, and may carry out necessary data manipulations.

Referring to Figure 3, a block diagram illustrating example control circuitry 230 is shown. It should be appreciated that Figure 3 illustrates a non-limiting example of a control circuitry 230 implementation. Other implementations of the control circuitry 230 not shown in Figure 3 are also possible. The control circuitry 230 may comprise a processor 310, a memory 320, and an input/output interface 330 connected with a bus 340. Under the control of the processor 310, data may be received from an external source through the input/output interface 330 and stored in the memory 320, and/or may be transmitted from the memory 320 to an external destination through the input/output interface 330. The processor 310 may process, add, remove, change, or otherwise manipulate data stored in the memory 320. Further, code may be stored in the memory 320. The code, when executed by the processor 310, may cause the processor 310 to perform operations relating to data manipulation and/or transmission and/or any other possible operations.

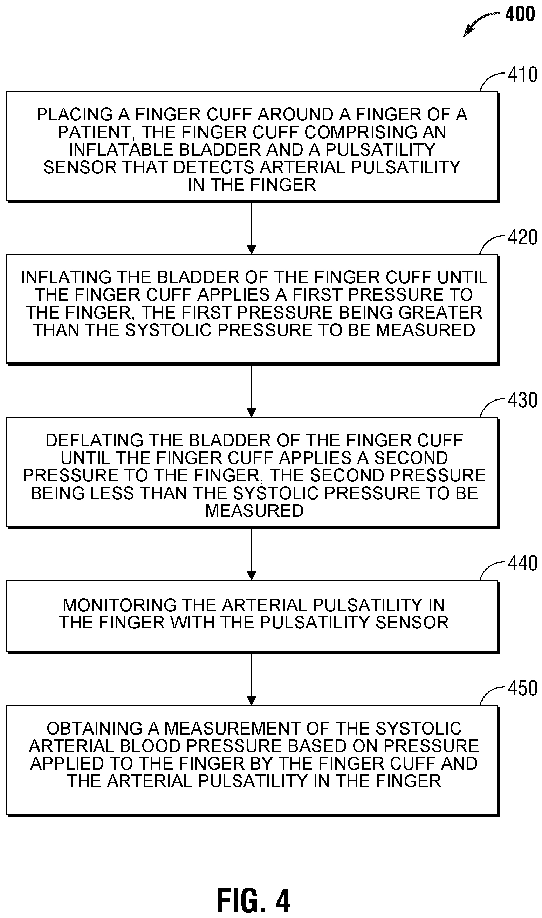

Referring to Figure 4, a flowchart illustrating an example method 400 for measuring a finger systolic arterial blood pressure is shown. At block 410, a finger cuff may be placed around a finger of a patient. The finger cuff may comprise an inflatable bladder and a pulsatility sensor that detects arterial pulsatility in the finger. At block 420, the bladder of the finger cuff may be inflated until the finger cuff applies a first pressure to the finger, the first pressure being greater than the systolic pressure to be measured. At block 430, the bladder of the finger cuff may be deflated until the finger cuff applies a second pressure to the finger, the second pressure being less than the systolic pressure to be measured. At block 440, the arterial pulsatility in the finger may be monitored with the pulsatility sensor. At block 450, a measurement of the finger systolic arterial blood pressure may be obtained based on pressure applied to the finger by the finger cuff and the arterial pulsatility in the finger. The measured finger systolic arterial blood pressure may be used to calibrate a continuous arterial blood pressure measurement system.

Further, as will be described, embodiments include measuring a pressure (e.g., a third pressure) applied to the finger by the finger cuff at a first time instant when the arterial pulsatility in the finger disappears when the bladder of the finger cuff is inflated to determine the finger systolic arterial blood pressure that may be used to calibrate the continuous arterial blood pressure measurement system. Additionally, embodiments include measuring a pressure (e.g., a fourth pressure) applied to the finger by the finger cuff at a second time instant when the arterial pulsatility in the finger resumes when the bladder of the finger cuff is deflated to determine the finger systolic arterial blood pressure that may be used to calibrate the continuous arterial blood pressure measurement system. Moreover, in one embodiment, an average of the third pressure and the fourth pressure may be used to determine the finger systolic arterial blood pressure that may be used to calibrate the continuous arterial blood pressure measurement system.

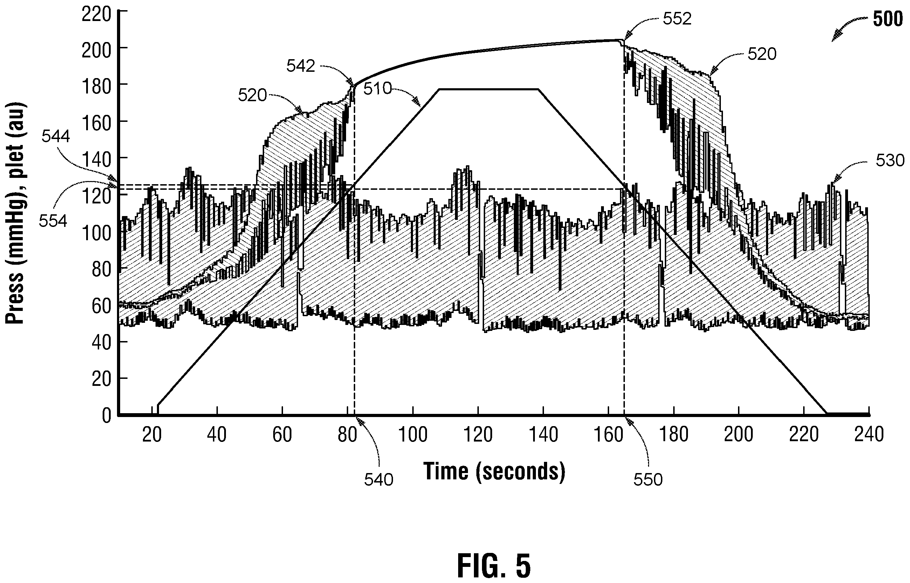

Referring to Figure 5, a diagram 500 illustrating various measurement traces according to embodiments is shown. Figure 5 shows that measurements obtained according to the example method 400 correspond to the systolic arterial pressure. Three measurement traces are shown in Figure 5: a first trace 510, a second trace 520, and a third trace 530. The first trace 510 shows the pneumatic pressure within the bladder 212, which corresponds to the pressure applied to the finger by the finger cuff 210. The second trace 520 shows the plethysmogram obtained from the pulsatility sensor 214 (e.g., a plethysmograph). Further, the third trace 530 shows the continuous blood pressure measured with the volume clamp method.

As can be seen in Figure 5, the bladder of the finger cuff may be inflated until the finger cuff applies a first pressure to the finger (trace 510) (e.g., above point 544), the first pressure being greater than the systolic pressure to be measured. Further, the bladder of the finger cuff may be deflated until the finger cuff applies a second pressure to the finger (trace 510) (e.g., below point 554), the second pressure being less than the systolic pressure to be measured.

In particular, when the bladder 212 is inflated (the rising segment of the first trace 510), at a first time instant 540, the pulsatility in the finger disappears due to the finger cuff pressure, which corresponds to the point 542 on the second trace 520 where oscillation stops, the pneumatic pressure 544 within the bladder 212 corresponds to the systolic arterial pressure. In this example, the pressure (e.g., the third pressure) is measured and may be used to calibrate the continuous arterial blood pressure measurement system. Similarly, when the bladder 212 is deflated (the falling segment of the first trace 510), at a second time instant 550 that the pulsatility in the finger resumes, which corresponds to the point 552 on the second trace 520 where oscillation reappears, the pneumatic pressure 554 within the bladder 212 also corresponds to the systolic arterial pressure. In this example, the pressure (e.g., the fourth pressure) is measured and may be used to calibrate the continuous arterial blood pressure measurement system. Further, in one embodiment, an average of these measured pressures (e.g., third and fourth pressures) may be used to determine the finger systolic arterial blood pressure that may be used to calibrate the continuous arterial blood pressure measurement system.

Therefore, according to the descriptions with reference to Figures 4 and 5, a finger systolic arterial blood pressure may be obtained by proper measurements and may be used to calibrate the continuous arterial blood pressure measurement system that operates according to the volume clamp method.

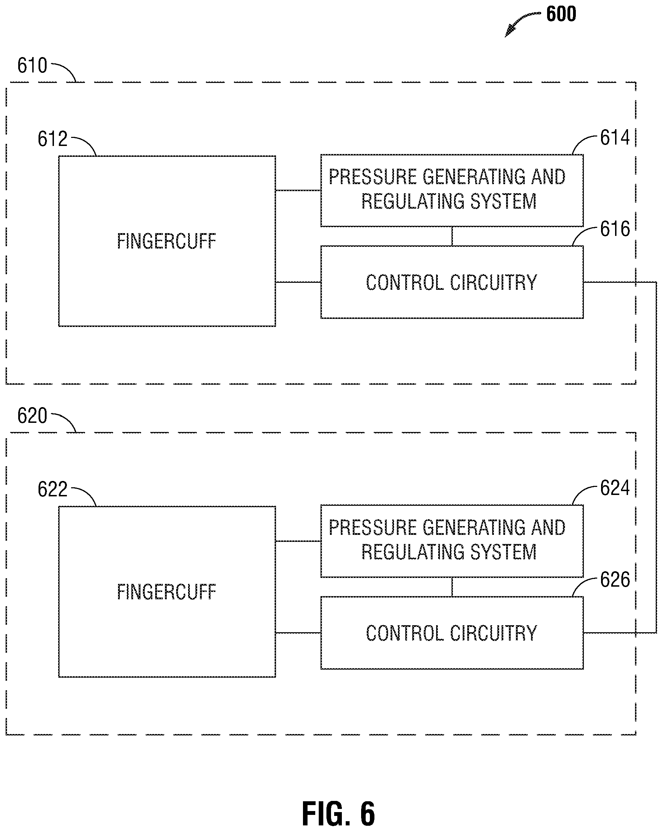

Referring to Figure 6, a block diagram illustrating an example environment 600 in which embodiments of the invention may be practiced is shown. The example environment 600 as illustrated in Fig. 6 is not claimed. The environment 600 may comprise two finger arterial blood pressure measurement systems each comprising a finger cuff, a pressure generating and regulating system, and control circuitry: the first system 610 comprises a finger cuff 612, a pressure generating and regulating system 614, and control circuitry 616, and the second system 620 comprises a finger cuff 622, a pressure generating and regulating system 624, and control circuitry 626. The control circuitry 616 of the first system 610 and the control circuitry 626 of the second system 620 may be connected so that data may be exchanged between the two systems. In one embodiment, the two systems 610, 620 may make finger arterial blood pressure measurements on the same patient. Thus, the finger cuffs 612, 622 may be placed on two separate fingers of the patient. In accordance with an embodiment of the invention, the first system 610 may make continuous arterial blood pressure measurements using the volume clamp method, while at the same time the second system 620 may make finger systolic arterial blood pressure measurements for calibration, as previously described. Thus, the measurements made by the second system 620 may be used to calibrate the first system 610 during running measurements in tandem.

In another embodiment of the invention, a single finger cuff may be used for both continuous arterial blood pressure measurement and calibration measurement. The continuous arterial blood pressure measurement may be performed with the volume clamp method, and the calibration measurement may be performed with the example method 400 previously described. The readings obtained from the calibration measurement may be utilized for calibration during continuous arterial blood pressure measurement. Therefore, the single finger cuff and the associated system including the associated pressure generating and regulating system may switch between the two modes.

It should be appreciated that because physiological changes may occur after a period of time in the finger on which continuous arterial blood pressure measurement is performed and the physiological changes may affect measurement accuracy, periodic recalibration may be required for the continuous arterial blood pressure measurement. The periodic recalibration may be performed with either the two-finger-cuff setup or the single-finger-cuff setup, as described herein. In one embodiment, where the two-finger-cuff setup is utilized, the roles (continuous measurement / calibration measurement) of the two finger cuffs and their associated systems may be switched from time to time.

It should be further appreciated that in repeating calibrations, the pressure range (e.g., the difference between the first pressure and the systolic pressure to be measured, and/or the difference between the second pressure and the systolic pressure to be measured) may be adjusted and preferably reduced based on the initial measurement and/or readings from the continuous arterial blood pressure measurement. A reduced pressure range may lead to a reduced calibration measurement time.

Therefore, embodiments of the invention provide a method to accurately obtain systolic values of the finger arterial pressure, which is beneficial, in that systolic pressure is currently the most difficult value to accurately obtain from the finger. The measurements made according to the embodiments of the invention may be used to calibrate running volume clamp-based blood pressure measurements in tandem such that running volume clamp-based blood pressure measurements are periodically or continuously updated. Thus, the calibration can be unobtrusively performed while blood pressure measurements continue on an adjacent finger. Moreover, no brachial cuff is needed for the calibration.

It should be appreciated that aspects of the invention previously described may be implemented in conjunction with the execution of instructions or code by processors, circuitry, controllers, control circuitry, etc. (e.g., processor 310 of Figure 3). As an example, control circuity may operate under the control of a program, algorithm, code, routine, or the execution of instructions to execute methods or processes (e.g., method 400 of Figure 4) in accordance with embodiments of the invention previously described. For example, such a program may be implemented in firmware or software (e.g. stored in memory and/or other locations) and may be implemented by processors, control circuitry, and/or other circuitry, these terms being utilized interchangeably. Further, it should be appreciated that the terms processor, microprocessor, circuitry, control circuitry, circuit board, controller, microcontroller, etc., refer to any type of logic or circuitry capable of executing logic, commands, instructions, software, firmware, functionality, etc., which may be utilized to execute the embodiments of the invention previously described.

The various illustrative logical blocks, processors, modules, and circuitry described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, non-transitory computer readable medium, or any other form of storage medium known in the art. An exemplary storage medium is coupled to the processor such the processor can read information from, and write information to, the storage medium. In the alternative, the storage medium may be integral to the processor.

## Claims

1. A method for monitoring blood pressure of a patient, comprising
- using a volume clamp method, continuously measuring a patient's arterial blood pressure with a continuous first arterial blood pressure measurement system (610);
- calibrating (400) the continuous first arterial blood pressure measurement system using a second arterial blood pressure measurement system (620) measuring systolic arterial blood pressure of the patient, the calibrating (400) comprising the consecutive steps of:
- placing (410) around a finger of the patient a finger cuff (622) that includes an inflatable bladder (212) and a pulsatility sensor (214) that detects arterial pulsatility in the finger of the patient;
- inflating (420) the bladder (212) of the finger cuff (622) until the finger cuff (622) applies a first pressure to the finger, the first pressure being greater than the systolic pressure to be measured;
- deflating (430) the bladder (212) of the finger cuff (622) until the finger cuff applies a second pressure to the finger, the second pressure being less than the systolic pressure to be measured;
- monitoring (440) the arterial pulsatility in the finger with the pulsatility sensor (214); and
- obtaining (450) a measurement of the systolic arterial blood pressure based on pressure applied to the finger by the finger cuff (622) and the arterial pulsatility in the finger; the method further comprising:
- using the measurement of the systolic arterial blood pressure obtained to calibrate the first arterial blood pressure measurement system; and
- the first arterial blood pressure measurement system and the second arterial blood pressure measurement system either using two different finger cuffs (612, 622) to continuously measure the patient's arterial blood pressure and to measure the systolic arterial blood pressure or using a single finger cuff, switching between two modes to continuously measure the patient's arterial blood pressure or to measure the systolic arterial blood pressure.

2. The method of claim 1, wherein obtaining (450) the measurement of the systolic arterial blood pressure further comprises measuring a third pressure applied to the finger by the finger cuff at a first time instant when the arterial pulsatility in the finger disappears when the bladder of the finger cuff (622) is inflated as the finger systolic arterial blood pressure.

3. The method of claim 2, wherein obtaining (450) the measurement of the systolic arterial blood pressure further comprises measuring a fourth pressure applied to the finger by the finger cuff at a second time instant when the arterial pulsatility in the finger resumes when the bladder of the finger cuff (622) is deflated as the finger systolic arterial blood pressure.

4. The method of claim 3, wherein obtaining (450) the measurement of the systolic arterial blood pressure further comprises measuring an average of the third pressure and the fourth pressure as the finger systolic arterial blood pressure.

5. The method of claim 1, wherein a pressure generating and regulating system, that is pneumatically connected to the inflatable bladder (212), generates, measures, and regulates pneumatic pressure.

6. The method of claim 1, wherein monitoring (440) the arterial pulsatility in the finger comprises using a plethysmograph (214), which comprises a light-emitting diode - photodiode pair.

7. The method of any of the preceding claims, wherein continuously measuring the patient's arterial blood pressure with the continuous first arterial blood pressure measurement system (610) comprises making continuous arterial blood pressure measurements using a finger arterial blood pressure measurement system comprising the finger cuff (612), a pressure generating and regulating system (614), and control circuitry (616).

8. The method of claim 7, wherein the finger cuffs (612, 622) of the first arterial blood pressure measurement system (610) and of the second arterial blood pressure measurement system (620) are placed around separate fingers of the patient.

9. The method of any of the preceding claims, wherein the second arterial blood pressure measurement system (620) calibrates the first arterial blood pressure measurement system (610) while at the same time the first arterial blood pressure measurement system is making continuous arterial blood pressure measurements.

10. A system for monitoring systolic arterial blood pressure of a patient, comprising:
- a first arterial blood pressure measurement system (610) for continuously measuring a patient's arterial blood pressure using a volume clamp method;
- a second arterial blood pressure measurement system (620) for measuring systolic arterial blood pressure of the patient, comprising
- a finger cuff including an inflatable bladder; and
- a pulsatility sensor for detecting arterial pulsatility in a finger of the patient, wherein the second arterial blood pressure measurement system (620) is configured to:
- inflate the bladder of the finger cuff until the finger cuff applies a first pressure to the finger when placed around the finger of the patient, the first pressure being greater than the systolic pressure to be measured;
- deflate the bladder of the finger cuff until the finger cuff applies a second pressure to the finger, the second pressure being less than the systolic pressure to be measured;
- monitor the arterial pulsatility in the finger with the pulsatility sensor; and to
- obtain a measurement of the systolic arterial blood pressure based on the pressure applied to the finger by the finger cuff and on the arterial pulsatility in the finger; wherein
- either the first arterial blood pressure measurement system and the second arterial blood pressure measurement system each comprise a respective one of two different finger cuffs (612, 622), or the system comprises a single finger cuff and is configured to switch between two modes to measure the systolic arterial blood pressure or to continuously measure the patient's arterial blood pressure, and wherein
the system is further configured to use the obtained measurement of the systolic arterial blood pressure to calibrate the first arterial blood pressure measurement system.

11. The system of claim 10, wherein the second arterial blood pressure measurement system (620) is configured to obtain the measurement of the systolic arterial blood pressure by further measuring as the finger systolic arterial blood pressure a third pressure applied to the finger by the finger cuff at a first time instant when the arterial pulsatility in the finger disappears when the bladder of the finger cuff is inflated.

12. The system of claim 10, wherein the inflatable bladder is pneumatically connected to a pressure generating and regulating system that generates, measures, and regulates pneumatic pressure.

13. The system of claim 10, wherein the pulsatility sensor comprises a plethysmograph.

14. The system of claim 10, wherein the second arterial blood pressure measurement system (620) is configured to calibrate the continuous first arterial blood pressure measurement system (610) while at the same time the first arterial blood pressure measurement system is making continuous arterial blood pressure measurements.

15. A non-transitory computer-readable medium comprising code which, when executed by a processor of a system for monitoring systolic arterial blood pressure of a patient according to any of claims 10 to 14, causes the processor to control performance of the method according to any of claims 1 to 9.

## Patentansprüche

1. Verfahren zur Überwachung des Blutdrucks eines Patienten, umfassend
- das kontinuierliche Messen des arteriellen Blutdrucks eines Patienten mit einem ersten kontinuierlichen arteriellen Blutdruckmesssystem (610) unter Verwendung eines Volumenklemmverfahrens;
- Kalibrieren (400) des ersten kontinuierlichen arteriellen Blutdruckmesssystems unter Verwendung eines zweiten arteriellen Blutdruckmesssystems (620), das den systolischen arteriellen Blutdruck des Patienten misst, wobei das Kalibrieren (400) die folgenden aufeinanderfolgenden Schritte umfasst:
- Legen (410) einer Fingermanschette (622) um einen Finger des Patienten, die eine aufblasbare Blase (212) und einen Pulsatilitätssensor (214) einschließt, der die arterielle Pulsatilität in dem Finger des Patienten erfasst;
- Aufblasen (420) der Blase (212) der Fingermanschette (622), bis die Fingermanschette (622) einen ersten Druck auf den Finger ausübt, wobei der erste Druck größer als der zu messende systolische Druck ist;
- Entlüften (430) der Blase (212) der Fingermanschette (622), bis die Fingermanschette einen zweiten Druck auf den Finger ausübt, wobei der zweite Druck geringer als der zu messende systolische Druck ist;
- Überwachen (440) der arteriellen Pulsatilität im Finger mit dem Pulsatilitätssensor (214); und
- Erhalten (450) einer Messung des systolischen arteriellen Blutdrucks auf Basis des ausgeübten Drucks der Fingermanschette (622) auf den Finger und der arteriellen Pulsatilität in dem Finger; das Verfahren ferner umfassend:
- Verwenden der erhaltenen Messung des systolischen arteriellen Blutdrucks zur Kalibrierung des ersten arteriellen Blutdruckmesssystems;
und
- des ersten arteriellen Blutdruckmesssystems und des zweiten arteriellen Blutdruckmesssystem unter Verwendung von entweder zwei verschiedenen Fingermanschetten (612, 622), um den arteriellen Blutdruck des Patienten kontinuierlich zu messen und um den systolischen arteriellen Blutdruck zu messen oder unter Verwendung von einer einzigen Fingermanschette, wobei zwischen zwei Modi umgeschaltet wird, um den arteriellen Blutdruck des Patienten kontinuierlich zu messen und um den systolischen arteriellen Blutdruck zu messen.

2. Verfahren nach Anspruch 1, wobei das Erhalten (450) der Messung des systolischen arteriellen Blutdrucks ferner das Messen eines dritten Drucks, der durch die Fingermanschette auf den Finger zu einem ersten Zeitpunkt ausgeübt wird, wenn die arterielle Pulsatilität im Finger verschwindet, wenn die Blase der Fingermanschette (622) aufgeblasen wird, als den systolischen arteriellen Blutdruck am Finger umfasst.

3. Verfahren nach Anspruch 2, wobei das Erhalten (450) der Messung des systolischen arteriellen Blutdrucks ferner das Messen eines vierten Drucks, der durch die Fingermanschette auf den Finger zu einem zweiten Zeitpunkt ausgeübt wird, wenn die arterielle Pulsatilität im Finger wiederbeginnt, wenn die Blase der Fingermanschette (622) entlüftet wird, als den systolischen arteriellen Blutdruck am Finger, umfasst.

4. Verfahren nach Anspruch 3, wobei das Erhalten (450) der Messung des systolischen arteriellen Blutdrucks ferner das Messen eines Durchschnitts des dritten Drucks und des vierten Drucks als den systolischen arteriellen Blutdruck am Finger umfasst.

5. Verfahren nach Anspruch 1, wobei ein Druckerzeugungs- und -regulierungssystem, das pneumatisch mit der aufblasbaren Blase (212) verbunden ist, den pneumatischen Druck erzeugt, misst und reguliert.

6. Verfahren nach Anspruch 1, wobei die Überwachung (440) der arteriellen Pulsatilität im Finger das Verwenden eines Plethysmographen (214) umfasst, der ein lichtemittierendes Diode-Fotodiodenpaar umfasst.

7. Verfahren nach einem der vorstehenden Ansprüche, wobei das kontinuierliche Messen des arteriellen Blutdrucks des Patienten mit dem ersten kontinuierlichen arteriellen Blutdruckmesssystem (610) das Durchführen kontinuierlicher arterieller Blutdruckmessungen unter Verwendung eines arteriellen Fingerblutdruckmesssystems umfasst, das die Fingermanschette (612), ein Druckerzeugungs- und -regulierungssystem (614) und einen Steuerschaltkreis (616) umfasst.

8. Verfahren nach Anspruch 7, wobei die Fingermanschetten (612, 622) des ersten arteriellen Fingerblutdruckmesssystems (610) und des zweiten arteriellen Blutdruckmesssystems (620) um separate Finger des Patienten gelegt werden.

9. Verfahren nach einem der vorstehenden Ansprüche, wobei das zweite arterielle Blutdruckmesssystem (620) das erste arterielle Blutdruckmesssystem (610) kalibriert, während zur gleichen Zeit das erste arterielle Blutdruckmesssystem kontinuierliche arterielle Blutdruckmessungen durchführt.

10. System zur Überwachung des systolischen arteriellen Blutdrucks eines Patienten, umfassend:
- ein erstes arterielles Blutdruckmesssystem (610) zur kontinuierlichen Messung des arteriellen Blutdrucks eines Patienten unter Verwendung eines Volumenklemmverfahrens;
- ein zweites arterielles Blutdruckmesssystem (620) zur Messung des systolischen arteriellen Blutdrucks des Patienten, umfassend
- eine Fingermanschette, die eine aufblasbare Blase einschließt; und
- einen Pulsatilitätssensor, um die arterielle Pulsatilität in einem Finger des Patienten zu erfassen, wobei das zweite arterielle Blutdruckmesssystem (620) ausgebildet ist, um
- die Blase der Fingermanschette aufzublasen, bis die Fingermanschette einen ersten Druck auf den Finger ausübt, wenn sie um den Finger des Patienten gelegt ist, wobei der erste Druck größer als der zu messende systolische Druck ist;
- die Blase der Fingermanschette zu entlüften, bis die Fingermanschette einen zweiten Druck auf den Finger ausübt, wobei der zweite Druck geringer als der zu messende systolische Druck ist;
- die arterielle Pulsatilität im Finger mit dem Pulsatilitätssensor zu überwachen; und um
- eine Messung des systolischen arteriellen Blutdrucks auf Basis des ausgeübten Drucks der Fingermanschette auf den Finger und der arteriellen Pulsatilität im Finger zu erhalten; wobei
- entweder das erste arterielle Blutdruckmesssystem und das zweite arterielle Blutdruckmesssystem jeweils eine entsprechende von zwei verschiedenen Fingermanschetten (612, 622) umfassen oder das System eine einzige Fingermanschette umfasst, und ausgebildet ist, um zwischen zwei Modi umzuschalten, um den systolischen arteriellen Blutdruck zu messen oder den arteriellen Blutdruck des Patienten kontinuierlich zu messen, und wobei das System ferner ausgebildet ist, um die erhaltene Messung des systolischen arteriellen Blutdrucks zu verwenden, um das erste arterielle Blutdruckmesssystem zu kalibrieren.

11. System nach Anspruch 10, wobei das zweite arterielle Blutdruckmesssystem (620) ausgebildet ist, um die Messung des systolischen arteriellen Blutdrucks dadurch zu erhalten, dass es außerdem als den systolischen arteriellen Blutdruck des Fingers einen dritten Druck misst, der durch die Fingermanschette zu einem ersten Zeitpunkt auf den Finger ausgeübt wird, wenn die arterielle Pulsatilität im Finger verschwindet, wenn die Blase der Fingermanschette aufgeblasen wird.

12. System nach Anspruch 10, wobei die aufblasbare Blase pneumatisch mit einem Druckerzeugungs- und -regulierungssystem verbunden ist, das den pneumatischen Druck erzeugt, misst und reguliert.

13. System nach Anspruch 10, wobei der Pulsatilitätssensor einen Plethysmographen umfasst.

14. System nach Anspruch 10, wobei das zweite arterielle Blutdruckmesssystem (620) ausgebildet ist, um das kontinuierliche erste arterielle Blutdruckmesssystem (610) zu kalibrieren, während zur gleichen Zeit das erste arterielle Blutdruckmesssystem kontinuierliche arterielle Blutdruckmessungen durchführt.

15. Nicht-transitorisches computerlesbares Medium, das einen Code umfasst, der, wenn er von einem Prozessor eines Systems zur Überwachung des systolischen arteriellen Blutdrucks eines Patienten nach einem der Ansprüche 10 bis 14 ausgeführt wird, den Prozessor veranlasst, die Durchführung des Verfahrens nach einem der Ansprüche 1 bis 9 zu steuern.

## Revendications

1. Procédé de surveillance de la tension artérielle d'un patient, comprenant
- à l'aide d'un procédé de serrage volumique, la mesure en continu de la tension artérielle d'un patient par un premier système de mesure en continu de la tension artérielle (610) ;
- l'étalonnage (400) du premier système de mesure en continu de la tension artérielle à l'aide d'un deuxième système de mesure de la tension artérielle (620) mesurant la tension artérielle systolique du patient, l'étalonnage (400) comprenant les étapes consécutives de :
- placement (410) autour d'un doigt du patient d'un doigtier (622) qui comprend une poche gonflable (212) et un capteur de pulsatilité (214) qui détecte une pulsatilité artérielle dans le doigt du patient ;
- gonflement (420) de la poche (212) du doigtier (622) jusqu'à ce que le doigtier (622) applique une première pression au doigt, la première pression étant supérieure à la pression systolique à mesurer ;
- dégonflement (430) de la poche (212) du doigtier (622) jusqu'à ce que le doigtier applique une deuxième pression au doigt, la deuxième pression étant inférieure à la pression systolique à mesurer ;
- surveillance (440) de la pulsatilité artérielle dans le doigt par le capteur de pulsatilité (214) ; et
- obtention (450) d'une mesure de la tension artérielle systolique sur la base de la pression appliquée au doigt par le doigtier (622) et de la pulsatilité artérielle dans le doigt ; le procédé comprenant en outre :
- l'utilisation de la mesure de la tension artérielle systolique obtenue pour étalonner le premier système de mesure de la tension artérielle ;
et
- le premier système de mesure de la tension artérielle et le deuxième système de mesure de la tension artérielle utilisant soit deux doigtiers différents (612, 622) pour mesurer en continu la tension artérielle du patient et pour mesurer la tension artérielle systolique, soit un doigtier unique, la commutation entre deux modes pour mesurer en continu la tension artérielle du patient ou pour mesurer la tension artérielle systolique.

2. Procédé selon la revendication 1, l'obtention (450) de la mesure de la tension artérielle systolique comprenant en outre la mesure d'une troisième pression appliquée au doigt par le doigtier à un premier instant temporel auquel la pulsatilité artérielle dans le doigt disparaît lorsque la poche du doigtier (622) est gonflée en tant que tension artérielle systolique du doigt.

3. Procédé selon la revendication 2, l'obtention (450) de la mesure de la tension artérielle systolique comprenant en outre la mesure d'une quatrième pression appliquée au doigt par le doigtier à un deuxième instant temporel auquel la pulsatilité artérielle dans le doigt reprend lorsque la poche du doigtier (622) est dégonflée en tant que tension artérielle systolique du doigt.

4. Procédé selon la revendication 3, l'obtention (450) de la mesure de la tension artérielle systolique comprenant en outre la mesure d'une moyenne de la troisième pression et de la quatrième pression en tant que tension artérielle systolique du doigt.

5. Procédé selon la revendication 1, un système de génération et de régulation de pression, qui est relié pneumatiquement à la poche gonflable (212), générant, mesurant et régulant la pression pneumatique.

6. Procédé selon la revendication 1, la surveillance (440) de la pulsatilité artérielle dans le doigt comprenant l'utilisation d'un pléthysmographe (214), qui comprend une paire de diode électroluminescente-photodiode.

7. Procédé selon l'une quelconque des revendications précédentes, la mesure en continu de la tension artérielle du patient par le premier système de mesure en continu de la tension artérielle (610) comprenant la réalisation de mesures en continu de la tension artérielle à l'aide d'un système de mesure de la tension artérielle de doigt comprenant le doigtier (612), un système de génération et de régulation de pression (614) et un circuit de commande (616).

8. Procédé selon la revendication 7, les doigtiers (612, 622) du premier système de mesure de la tension artérielle de doigt (610) et du deuxième système de mesure de la tension artérielle (620) étant placés autour de doigts séparés du patient.

9. Procédé selon l'une quelconque des revendications précédentes, le deuxième système de mesure de la tension artérielle (620) étalonnant le premier système de mesure de la tension artérielle (610) tandis que, en même temps, le premier système de mesure de la tension artérielle effectue des mesures en continu de la tension artérielle.

10. Système de surveillance de la tension artérielle systolique d'un patient, comprenant :
- un premier système de mesure de la tension artérielle (610) pour mesurer en continu la tension artérielle d'un patient à l'aide d'un procédé de serrage volumique ;
- un deuxième système de mesure de la tension artérielle (620) pour mesurer la tension artérielle systolique du patient, comprenant
- un doigtier comprenant une poche gonflable ; et
- un capteur de pulsatilité pour détecter une pulsatilité artérielle dans un doigt du patient, le deuxième système de mesure de la tension artérielle (620) étant conçu pour :
- gonfler la poche du doigtier jusqu'à ce que le doigtier applique une première pression au doigt lorsqu'il est placé autour du doigt du patient, la première pression étant supérieure à la pression systolique à mesurer ;
- dégonfler la poche du doigtier jusqu'à ce que le doigtier applique une deuxième pression au doigt, la deuxième pression étant inférieure à la pression systolique à mesurer ;
- surveiller la pulsatilité artérielle dans le doigt par le capteur de pulsatilité ; et
- obtenir une mesure de la tension artérielle systolique sur la base de la pression appliquée au doigt par le doigtier et de la pulsatilité artérielle dans le doigt ;
- soit le premier système de mesure de la tension artérielle et le deuxième système de mesure de la tension artérielle comprenant chacun un doigtier respectif parmi deux doigtiers différents (612, 622), soit le système comprenant un doigtier unique et étant conçu pour commuter entre deux modes pour mesurer la tension artérielle systolique ou pour mesurer en continu la tension artérielle du patient et le système étant en outre conçu pour utiliser la mesure obtenue de la tension artérielle systolique pour étalonner le premier système de mesure de la tension artérielle.

11. Système selon la revendication 10, le deuxième système de mesure de la tension artérielle (620) étant conçu pour obtenir la mesure de la tension artérielle systolique en mesurant en outre, en tant que tension artérielle systolique de doigt, une troisième pression appliquée au doigt par le doigtier à un premier instant temporel lorsque la pulsatilité artérielle dans le doigt disparaît lorsque la poche du doigtier est gonflée.

12. Système selon la revendication 10, la poche gonflable étant reliée pneumatiquement à un système de génération et de régulation de pression qui génère, mesure et régule la pression pneumatique.

13. Système selon la revendication 10, le capteur de pulsatilité comprenant un pléthysmographe.

14. Système selon la revendication 10, le deuxième système de mesure de la tension artérielle (620) étant conçu pour étalonner le premier système de mesure en continu de la tension artérielle (610) tandis que, en même temps, le premier système de mesure de la tension artérielle effectue des mesures en continu de la tension artérielle.

15. Support non transitoire lisible par ordinateur comprenant un code qui, lorsqu'il est exécuté par un processeur d'un système de surveillance de la tension artérielle systolique d'un patient selon l'une quelconque des revendications 10 à 14,
amène le processeur à commander les performances du procédé selon l'une quelconque des revendications 1 à 9.
